# EUROPEAN PATENT SPECIFICATION

(11) **EP 4 312 791 B1**
(45) Date of publication and mention of the grant of the patent: **23.10.2024**
(21) Application number: 22717089.1
(22) Date of filing: 21.03.2022
(51) Int. Cl.: A61B 8/00

(54) **REAL-TIME ON-CART CLEANING AND DISINFECTING GUIDANCE TO REDUCE CROSS INFECTION AFTER ULTRASOUND EXAMINATION**
ECHTZEIT-ON-KART-REINIGUNGS- UND DESINFEKTIONSFÜHRUNG ZUR VERRINGERUNG VON KREUZINFEKTIONEN NACH EINER ULTRASCHALLUNTERSUCHUNG
GUIDAGE EN TEMPS RÉEL DE NETTOYAGE ET DE DÉSINFECTION DE CHARIOT PERMETTANT DE RÉDUIRE UNE INFECTION CROISÉE APRÈS EXAMEN PAR ULTRASONS

(30) Priority: 24.03.2021 US 202163165341 P
(43) Date of publication of application: 07.02.2024
(73) Proprietor: Koninklijke Philips N.V., 5656 AG Eindhoven (NL)
(72) Inventor: XIE, Hua, 5656 AG Eindhoven (NL); ERRICO, Claudia, 5656 AG Eindhoven (NL); SADEGHI, Ali, 5656 AG Eindhoven (NL); NGUYEN, Man, 5656 AG Eindhoven (NL); KRUECKER, Jochen, 5656 AG Eindhoven (NL); PREVRHAL, Sven Peter, 5656 AG Eindhoven (NL)
(74) Representative: Philips Intellectual Property & Standards
(86) International application number: PCT/EP2022/057262
(87) International publication number: WO 2022/200238

(56) References cited:
- US-A1- 2008 188 754
- US-A1- 2018 250 428
- CARRICO RUTH M ET AL: "Ultrasound probe use and reprocessing: Results from a national survey among U.S. infection preventionists", AJIC: AMERICAN JOURNAL OF INFECTION CONTROL, ELSEVIER, AMSTERDAM, NL, vol. 46, no. 8, 1 June 2018 (2018-06-01), pages 913 - 920, XP085431942, ISSN: 0196-6553, DOI: 10.1016/J.AJIC.2018.03.025

## Description

### FIELD OF THE INVENTION

The invention relates to the field of ultrasound imaging and more particularly to identifying disinfection requirements for an ultrasound system between procedures and notifying a user of the disinfection requirements.

### BACKGROUND

Ultrasound is the most widely used medical imaging modality in the world because it is real-time, mobile, and affordable. With continuous improvement and recent advances in ultrasound technology, ultrasound has attracted a broad array of clinical applications and different users in traditional radiology departments, cardiology departments, obstetrics/gynecology centers, and new emerging clinical areas such as emergency departments, interventional suites, and point of care settings, such as primary care offices and body check-up centers.

In any ultrasound procedure, ultrasound probes will be in contact with a patient's body and tissue in various fashions. Transducers can come in contact with a patient's blood, a patient's mucosal surfaces (such as digestive tract, urinary tract, and respiratory tract), biopsy sites, and/or wounds. Patients may be infected with hepatitis B and C and recently COVID-19. In such environments bacteria, pathogens, and viruses can be transmitted between procedures and pose significant risks to ultrasound practitioners and patients without proper infection prevention and disinfection practices.

Based on the risk level of cross-infection, ultrasound procedures are typically classified into one of three Spaulding categories according to the Centers for Disease Control (CDC) guidelines.
1) Non-invasive, non-critical: transducer in contact with intact, healthy tissue, for example, normal transabdominal obstetric (OB) exam, thoracic cardiac imaging, transabdominal liver imaging, and breast imaging.
2) Semi-critical: transducer with protective cover in contact with mucous membrane, such as transvaginal exam, transrectal exam, and transesophageal cardiac exam.
3) Critical and sterile: transducer contacts sterile or vascular tissue or an interventional device (e.g., needle, catheter) inserted into sterile tissue or body cavity during a procedure, such as ultrasound guided aspiration, ultrasound guided biopsy, ultrasound guided surgery, percutaneous radiofrequency ablation, etc.

Government healthcare agencies such as the CDC, and Food and Drug Administration (FDA) have developed general guidelines for decontaminating medical devices. Ultrasound organizations worldwide have also released more specific guidelines for cleaning ultrasound probes and systems between ultrasound procedures to reduce cross-contamination, including American Institute for Ultrasound Medicine, American College of Emergency Physicians, The Society of Diagnostic Medical Sonography, World Federation for Ultrasound in Medicine and Biology, European Society of Radiology, and European Committee for Medical Ultrasound Safety.

Depending on the procedure type, three levels of disinfection are required by the guidelines. Table 1 maps the procedure type to the required disinfection process. The three levels of disinfection are:
1) Low-Level Disinfection (LLD) will destroy most bacteria, some viruses, and some fungi. LLD comprises the use of i. soap and water and/or ii. quarternary ammonia sprays or wipes. LLD is typically performed manually.
2) High-Level Disinfection (HLD) removes all microorganisms except for bacteria spores, unless used under specialized conditions. HLD comprises the use of i) chemical sterilants or germicides and/or ii) physical sterilization. HLD is performed manually or automatically.
3)Sterilization removes final residues of bacteria spores. Sterilization uses i) ethylene oxide gas and/or ii) hydrogen peroxide plasma. To guarantee the efficacy and reliability of sterilization, sterilization temperature, pressure, humidity, and exposure time must meet certain requirements. The heat and pressure requirements render some heat-sensitive or pressure-sensitive transducers unable to withstand sterilization. In these cases, transducers must be placed in a sterile cover with sterile gel during the imaging procedure and HDL performed post exam.

**TABLE 1**

| Spaulding Classification | Disinfection Level |
|---|---|
| Non-Critical | Cleaning + low-level disinfection |
| Semi-Critical | Cleaning + high-level disinfection |
| Critical | Cleaning + sterilization (if sterilization is not possible, then use high-level disinfection and a sterile transducer cover) |

Ultrasound system operators must closely inspect a patient's skin conditions for evidence of unhealthy tissue during ultrasound procedures. Ultrasound system operators must also inspect protective covers, if used, for evidence of leaks during the ultrasound procedure. If bodily fluid or blood is contacted during a non-critical procedure, it can change from a non-critical to a semi-critical to a critical procedure which calls for adjusting the post procedure disinfection process.

While government healthcare regulatory organization and healthcare industry organization guidelines focus on disinfecting ultrasound transducers, infection prevention and control principles are also important to the ultrasound scanner along with any ancillary equipment used during an ultrasound procedure (e.g., touch screen, control panel, EEG cables, scanner monitor).

US 2008/188754 describes an ultrasound probe having an operational indicator assembly for indicating operational data relating to the ultrasound probe, including data relating to a sterility of the ultrasound probe.

### SUMMARY

The inventors of the present invention have realized that it would be advantageous to provide an ultrasound system that identifies recommended post-exam disinfection and cleaning steps for an ultrasound system and provides an alert of the recommended disinfection and cleaning steps to a user of the ultrasound system.

According to a first aspect of the present invention, a computer-implemented method is provided for identifying recommended post-exam disinfection and cleaning steps for an ultrasound system and providing an alert of said recommended disinfection and cleaning steps to a user of the ultrasound system. The method comprises the steps of:
obtaining clinical informatics, examination details, user-selected ultrasound exam settings, and ultrasound images and annotations;
determining a recommended disinfection classification based on the clinical informatics, examination details, user-selected ultrasound exam settings, and ultrasound images and annotations and
providing an alert to a user to conduct a post exam disinfection procedure; the alert including the recommended disinfection classification.

According to one embodiment, the clinical informatics are obtained from an Electronic Health Record (EHR) system or an Electronic Medical Record (EMR) system, the examination details are obtained from an exam order form, and the user-selected exam settings and ultrasound images and annotations are obtained from an ultrasound imaging module.

According to one embodiment, the alert further includes a button for providing visual guidance for a disinfection protocol.

According to one embodiment, the method further comprises the steps of:
surveying a system service log file of the ultrasound system to determine user contact data for ultrasound system equipment surfaces; and
determining disinfection requirements for the ultrasound equipment system surfaces based on the user contact data;
wherein the visual guidance includes a heat map indicating user contacts with the ultrasound system equipment surfaces.

According to one embodiment the method further comprises the step of analyzing video data from at least one camera to determine user contact data for ultrasound system equipment. surfaces.

According to one embodiment the heat map is displayed as an image on a display of the ultrasound system.

According to one embodiment the heat map is displayed by backlighting contacted surfaces of the ultrasound system equipment.

According to one embodiment the alert includes a "proceed to disinfection" user interaction and a "done" user interaction, further comprising the step of providing disinfection protocol quality control by tracking an ultrasound transducer and a time duration between the timestamps for "proceed to disinfection protocol" and "done" user interactions.

According to another aspect of the present invention, an ultrasound system is provided that is configured to identify recommended post-exam disinfection and cleaning steps for the ultrasound system and providing an alert of the recommended disinfection and cleaning steps to a user of the ultrasound system, the system comprises: a processor, a memory operably connected to the processor; and a medical record system operably connected to the processor, wherein the memory has encoded thereon a program of instruction executable by the processor to perform the steps of:
obtaining clinical informatics, examination details, user-selected ultrasound exam settings, and ultrasound images and annotations;
determining a recommended disinfection classification based on the clinical informatics, examination details, user-selected ultrasound exam settings, and ultrasound images and annotations and
providing an alert to a user to conduct a post exam disinfection procedure; the alert including the recommended disinfection classification.

According to one embodiment, the alert further includes a button for providing visual guidance for a disinfection protocol.

According to one embodiment, the ultrasound system further comprises program code for performing the steps of:
surveying a system service log file of the ultrasound system to determine user contact data for ultrasound system equipment surfaces; and
determining disinfection requirements for the ultrasound equipment system surfaces based on the user contact data;
wherein the visual guidance includes a heat map indicating user contacts with the ultrasound system equipment surfaces.

According to one embodiment, the heat map is displayed as an image on a display of the ultrasound system.

According to one embodiment the heat map is displayed by backlighting contacted surfaces of the ultrasound system equipment.

According to another aspect of the present invention, a computer program product is provided comprising a machine-readable storage media having encoded thereon program code for identifying recommended post-exam disinfection and cleaning steps for an ultrasound system and providing an alert of the recommended disinfection and cleaning steps to a user of the ultrasound system, comprising:
program code for obtaining clinical informatics, examination details, user-selected ultrasound exam settings, and ultrasound images and annotations;
program code for determining a recommended disinfection classification based on the clinical informatics, examination details, user-selected ultrasound exam settings, and ultrasound images and annotations and
program code for providing an alert to a user to conduct a post exam disinfection procedure; the alert including the recommended disinfection classification.

According to another aspect of the present invention, a computer program product is provided for
The invention may be realized by software stored on a memory and executed by a processor operably connected to the memory. The predicted interpretation time may be presented on a display operably connected to the processor in a variety of data formats through any known connection method using any known communication protocol.

The term "processor", when used herein shall mean a single processor or a plurality of processors that may be interconnected through hardwiring or wireless connection or may be in communication through a network. The processors may be single-core or multi-core processors.

The term "memory", when used herein, shall mean a machine-readable medium that is either integral with the processor, such as in a workstation or general-purpose computer, or external to processor, such as an external hard drive, cloud storage, or a removable memory device. The medium can be an electronic, magnetic, optical, electromagnetic, infrared, or semiconductor system (or apparatus or device). Examples of a computer-readable medium include a semiconductor or solid-state memory, magnetic tape, a removable computer diskette, a random access memory (RAM), a read-only memory (ROM), a rigid magnetic disk, and an optical disk. Current examples of optical disks include compact disk-read only memory (CD-ROM), compact disk-read/write (CD-R/W) and DVD.

The term "display", when used herein, shall mean a human viewable computer interface for presenting image data or streams with or without additional images or data as stationary or moving pictures connected to the processor via video graphics array (VGA), digital visual interface (DVI), high-definition multimedia interface (HDMI), low-voltage differential signaling (LVDS) or other proprietary connectors and signals. Examples of currently used displays include liquid crystal displays, light emitting diode displays, plasma displays.

The term "and/or", when used herein, shall mean only the first possibility, only the second possibility, only the third possibility, and so forth as well as any combination of the listed possibilities. For example, the phrase A, B, and/or C can be any of: only A, only B, only C, A and B, A and C, B and C, or A, B, and C.

The term "clinical informatics", when used herein shall mean health information derived from an Electronic Health Record (EHR) system or an Electronic Medical Record (EMR) system, such as patient health metrics, medical conditions, diseases, and any other health or medical information useful to medical professionals.

The term "deep learning", when used herein shall mean an artificial intelligence algorithm comprising computer code that can learn to perform functions (such as identifying structures in ultrasound images) bassed on artificial neural networks with representation learning.

### BRIEF DESCRIPTION OF THE DRAWINGS

The features and advantages of the invention will be more clearly understood from the following detailed description of the preferred embodiments when read in connection with the accompanying drawing. Included in the drawing are the following figures:
Fig. 1 is a process flow for an ultrasound system for identifying disinfection recommendations and providing disinfection guidance to a user according to an embodiment of the invention;
Fig. 2 is a block diagram of an ultrasound system for identifying post-exam disinfection requirements and providing disinfection guidance to a user according to an embodiment of the invention;
Fig. 3 is a process flow for a process for determining an appropriate post-exam disinfection level according to an embodiment of the invention;
Figs. 4a and 4b show a medical record and an ultrasound order form respectively used for extracting disinfection related information according to an embodiment of the invention;

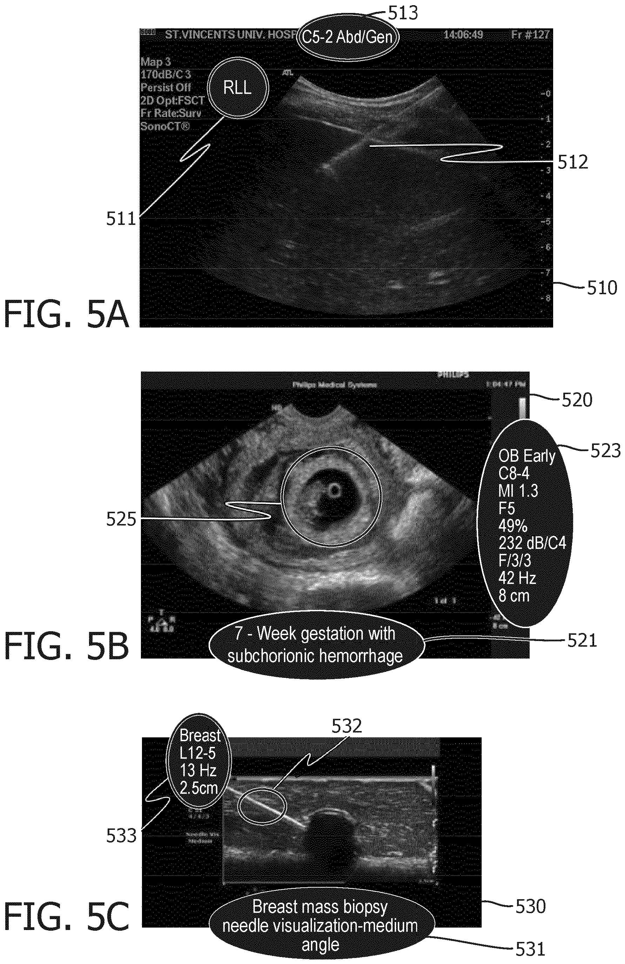

Figs. 5a-5c are ultrasound images showing image data used in embodiments of the present invention;
Fig. 6 is a flow diagram for a generating a heat map of user interface utilization for identifying user contact and associated disinfection requirements according to an embodiment of the invention;
Figs. 7a-7d show sources for contact information for identifying user contact and associated disinfection requirements according to an embodiment of the invention;
Fig. 8 is a flow diagram of a process for providing visual disinfection guidance to a user according to an embodiment of the present invention;
Fig. 9 shows a contact heat map for disinfection guidance according to an embodiment of the invention;
Fig. 10 shows an ultrasound system with backlit surfaces showing user contact for disinfection guidance according to an embodiment of the present invention; and
Fig. 11 shows a disinfection alert window providing a disinfection reminder nd disinfection guidance to a user according to an embodiment of the present invention.

### DETAILED DESCRIPTION

The inventors have identified a need for an ultrasound system that identifies disinfection requirements for an ultrasound system and provides real-time disinfection guidance to a user of the ultrasound system. To meet this need, the inventors have invented an intelligent on-cart decision support tool that integrates ultrasound imaging, system utilization, and clinical informatics to automatically remind and enforce cleaning and disinfecting procedures between exams.

In one embodiment, the invention comprises three sequential building blocks. It should be understood, however, that the invention may be practiced with less than all of the building blocks. Following is a brief description of the three building blocks. They will be described in greater detail below.

In the first building block, the invention obtains clinical informatics (e.g.patient medical records and/or an ultrasound exam order form), ultrasound exam settings selected by a user, and ultrasound images and annotations to classify a current exam into one of a plurality of disinfection classes (e.g., sterilization, HLD, LLD). The first building block leverages artificial intelligence (e.g., deep learning) and anatomical intelligence to support organ detection and interventional device detection.

In the second building block, the invention analyzes a system service log file and images captured by video cameras in the exam room or embedded on the scanner to detect user interface utilization during the exam and generated contact heatmaps for auxiliary and peripheral devices such as a touch screen, a control panel, a keyboard, a scanner monitor, and other equipment surfaces. The invention safeguards patient privacy when using cameras for contact tracking.

In the third building block. The invention provides guidance to a user to conduct a disinfection procedure after finishing the exam. The invention highlights at-risk areas using heatmaps on the scanner with a hazard warning light and advises the appropriate follow-up disinfection procedure.

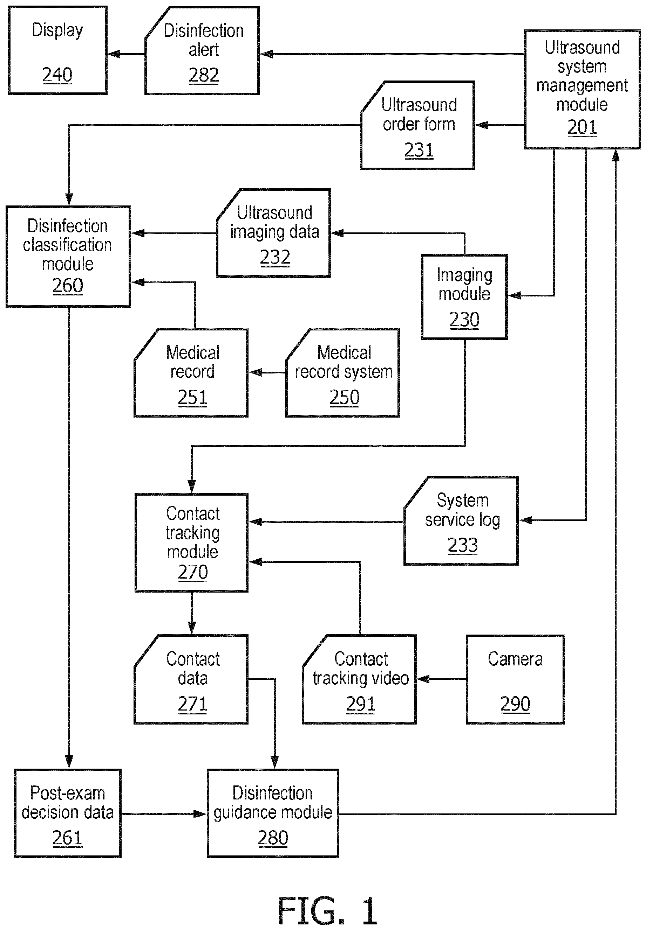

Fig. 1 illustrates the process flow for an ultrasound system for identifying disinfection requirements for an ultrasound system between procedures and providing disinfection guidance to a user according to an embodiment of the invention.

Prior to an ultrasound exam, a disinfection classification module 260 obtains a medical record 251 from a medical record system 250. The medical record system may be an Electronic Health Record (EHR) system or clinical management system or any other system that stores patient medical history. The medical record typically comprises a plethora of medical information associated with a current patient, often from a variety of medical practices and/or hospitals. Commercially available EHR platforms are designed to share medical information, and the medical record may be obtained using any known medical communication protocol following specifications and standards such as Fast Hospital Interoperability Resources (FHIR), digital imaging communication in medicine (DICOM), or the like.

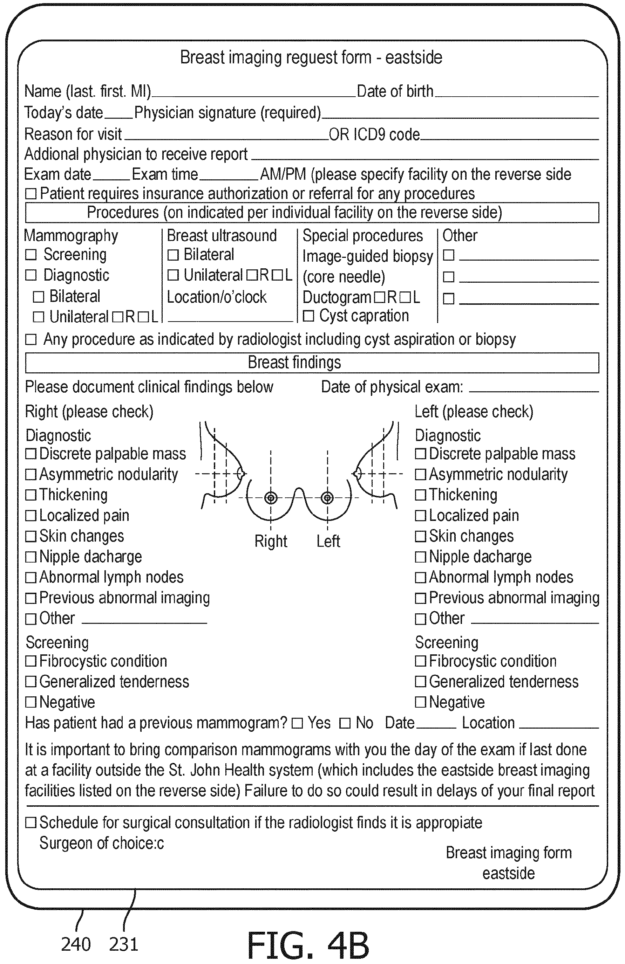

The disinfection classification module 260 also obtains an ultrasound order form 231 (shown in Fig. 4B) prior to the ultrasound examination. The ultrasound order form may be obtained from an ultrasound system management module 201 of the ultrasound system 200, itself. Alternatively, the ultrasound order form may be obtained from a clinical management system or any other source that is used to electronically complete an ultrasound order. The ultrasound order form 231 contains information about the procedure that is being performed and may include information relevant to post-exam or post-procedural cleaning and disinfection requirements. Like the medical record, the ultrasound order form may be obtained using any known medical communication protocol.

During the ultrasound examination, the disinfection classification module 260 obtains ultrasound imaging data 232 from an imaging module 230 of the ultrasound imaging system 200. The ultrasound imaging data 232 may comprise ultrasound images, tracking data derived from ultrasound images, annotations from medical professionals, probe identification and Tisue Specific Presets (TSP) data. The tracking data may be tracking the relative position of a probe or other device relative to an anatomical structure, such as a bodily organ. The annotations may be saved in a system service log file 233. TSP data is indicative of organs to be imaged, which can provide information about the invasiveness of the procedure which affects cleaning and disinfection requirement, as well as information useful in organ and instrument tracking.

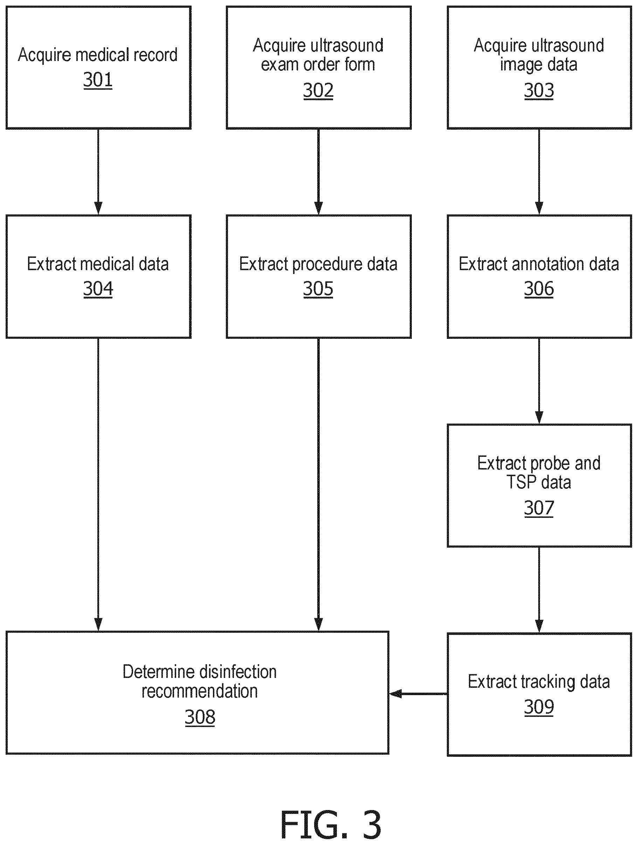

The disinfection classification module 260 processes the data from the medical record 251, the ultrasound order form 231, and the ultrasound imaging data 232 to determine post examination disinfection guidance 261 as will be presented in greater detail with reference to Fig. 3.

A contact tracking module 270 obtains the system service log entries 233 from an imaging module 230 of the ultrasound imaging system 200. The system service log 233 records each user input via user interfaces, such as a touch screen, control panel. The user inputs may be soft or hard button presses, such as: Time Gain Compensation (TGC) sliding, image freeze/unfreeze, "acquire" buttons, trackball movement, labeling through soft/hard keyboard, pre-defined annotation and body marker selection, Electrocardiogram (ECG) signal synchronization, moving the system by handrail, etc. Each user input via a user interface is listed in the service log file in chronological order as an individual event with starting and ending timestamps, which enables user activity retrieval for contact tracing.

In one embodiment, the contact tracking module 270 also obtains contact tracking video 291 from one or more cameras 290 mounted in the exam room and/or on an ultrasound scanner (e.g. built-in front-facing or rear-facing camera). The images can be used to capture user contact with surfaces of user interfaces and ancillary equipment.

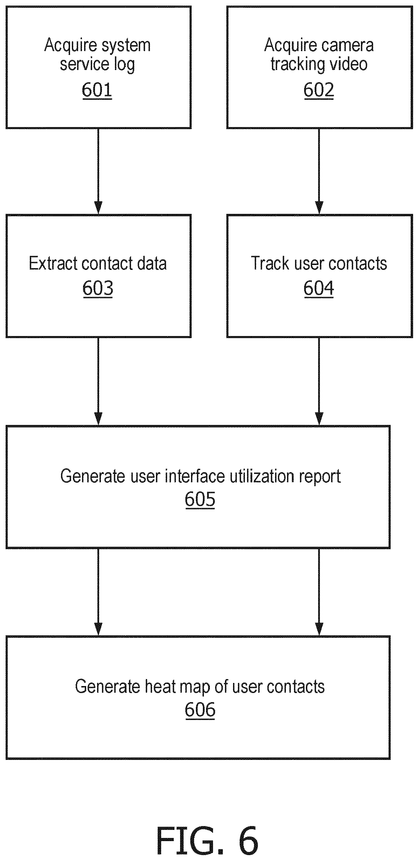

The contact tracking module 270 processes the contact data from the system service log 233 and the contact tracking video 291 to identify at-risk areas for disinfection guidance. This processing will be discussed in greater detail with reference to Fig. 6.

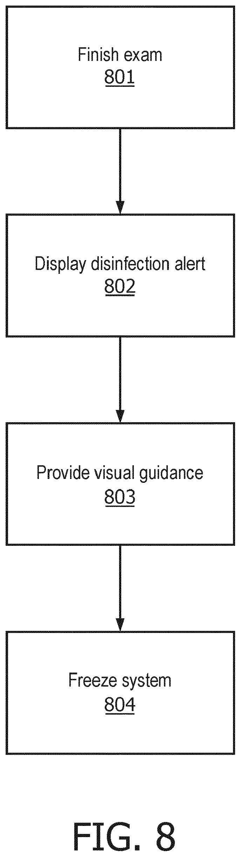

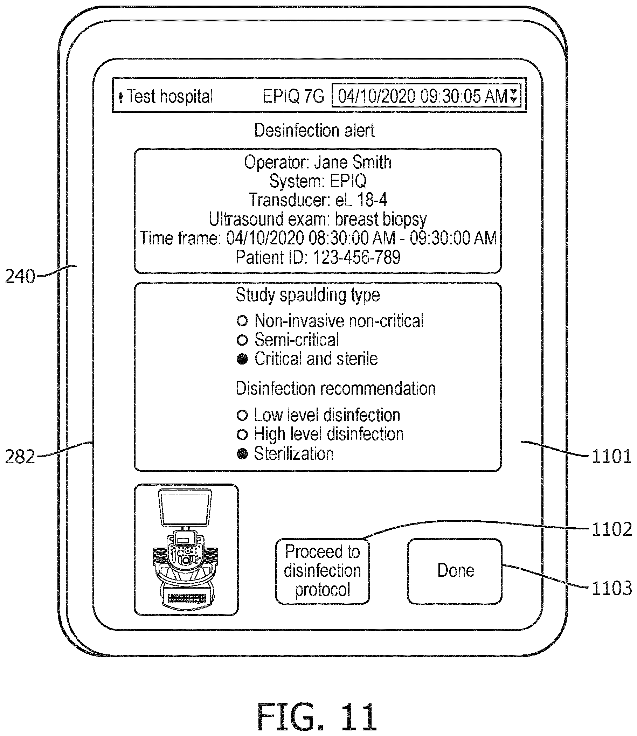

In existing ultrasound systems, the ultrasound operator will complete an exam, by pressing an "end exam" button on the scanner, for example. Then, the system will automatically display a new patient data entry window, and the operator will enter patient data and start a new exam. In an embodiment of the present invention, instead of displaying a new patient data entry window in response to completing an exam, the system will display a disinfection alert window 282 to remind a user or operator to disinfect the system prior to starting another exam and to provide disinfection guidance to the user. The process for providing the disinfection alert window and disinfection guidance will be described in greater detail with respect to Figs. 8-10. The disinfection alert window will be described in greater detail with respect to Fig. 11.

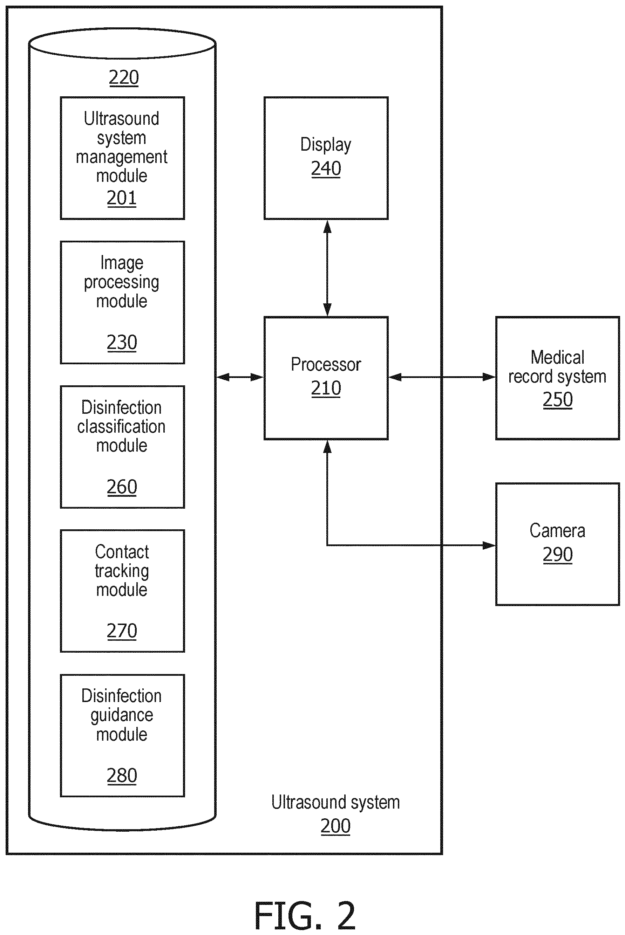

Fig. 2 shows an ultrasound system 200 incorporating disinfection requirement identification and disinfection guidance according to an embodiment of the invention. In the exemplary embodiment that follows, the ultrasound system 200 is a cart-based ultrasound system. However, the invention may be practiced with any ultrasound system.

The system comprises a processor 210 which executes program code to provide clinical management data in various formats and at various locations. The processor 210 is operably connected to a memory 220, which comprises a machine-readable media with program code and data stored thereon. Specifically, an ultrasound system management module 201, an image processing module 230, a disinfection classification module 260, a contact tracking module 270 and a disinfection guidance module 280 are stored on the memory 220. The ultrasound system management module 201, the image processing module 230, the disinfection classification module 260, the contact tracking module 270, and the disinfection guidance module may be stored on the same memory or on multiple memories.

The processor 210 is also operably connected to a display 240 which provides a visual user interface for a user to view imaging and for the system to provide other information to a user.

The imaging module 230 is operably connected to the processor 210 to provide imaging data such as images and/or loops of images to the processor 210 so that they can be made available to a sonographer and cardiologist for measurement and interpretation.

A medical record system 250 is also operably connected to the processor 210. While the medical record system 250 is shown as being directly connected to the processor 210, it should be understood that the medical record system 250 may also be connected to the processor through a network. The medical record system may be any electronic health record (EHR) system or electronic medical record (EMR) system, or a clinical management system, or any other source of electronic medical or health data.

In some embodiments of the invention, one or more cameras 290 are also operably connected to the processor 210 to provide contact tracking video 291 for use in determining user contacts with ultrasound system surfaces to provide disinfection guidance to the user based on the user contacts.

Fig. 3 is a process flow of the first building block of the invention - the disinfection classification module. This process 300 determines a recommended disinfection level based on evidence collected from various sources. The evidence includes clinical informatics (e.g., patient medical record and exam order form), ultrasound exam settings selected by the user, and ultrasound images and annotations. The process is performed by processor 210 executing the disinfection classification module 260. The disinfection classification module acquires a patient medical record (step 301) from a medical record system 250.

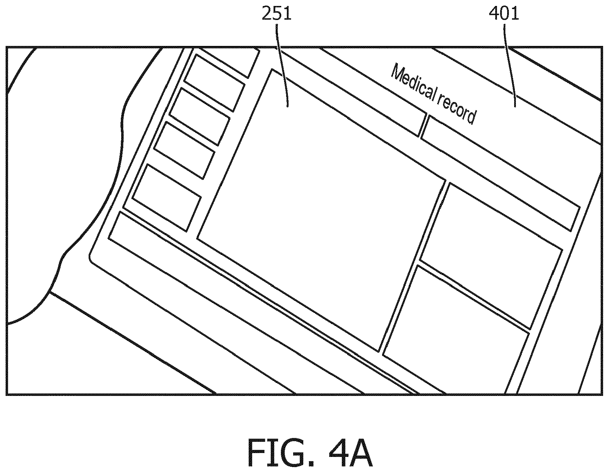

The disinfection classification module 260 processes the medical record 251 to extract medical data affecting disinfection requirements (step 304) for the ultrasound system. The medical data may include data such as a skin infection or a history of diseases like Hepatitis B, Hepatitis C, or COVID-19. The medical record may be processed using natural language processing, for example. As shown in Fig. 4A, the medical record 251 may be acquired on a handheld scanner 401 of an ultrasound system through a wireless connection. Alternatively, the medical record 251 may be acquired using a scanner mounted on an ultrasound cart or any other processing device operably connected to the medical record system 250.

The disinfection classification module 260 acquires the ultrasound exam order form 231 (step 302) from the ultrasound imaging system 200. The ultrasound exam order form 231 may be acquired from the ultrasound system management module 201 in the ultrasound scanner 401 or any other processing device operably connected to the ultrasound scanner. The order form 231 is stored in the ultrasound system memory 220 and displayed on display 240 or another device display. The ultrasound exam order form is filled out by a user through a user interface such as a keyboard.

The disinfection classification module 260 extracts relevant data from the ultrasound exam order form (step 305). This relevant data may include organ detection and intervention procedure detection requirements using natural language processing, which can then inform organ and interventional device tracking during the exam. For example, the ultrasound order form may define an organ and interventional device to be tracked. The relevant data may also include details of the ordered exam that affect the criticality of the exam, and therefore, the recommended disinfection classification. For example, an exam that requires transducer contact with mucosal surfaces would have a higher disinfection classification than an exam that only requires transducer contact with skin.

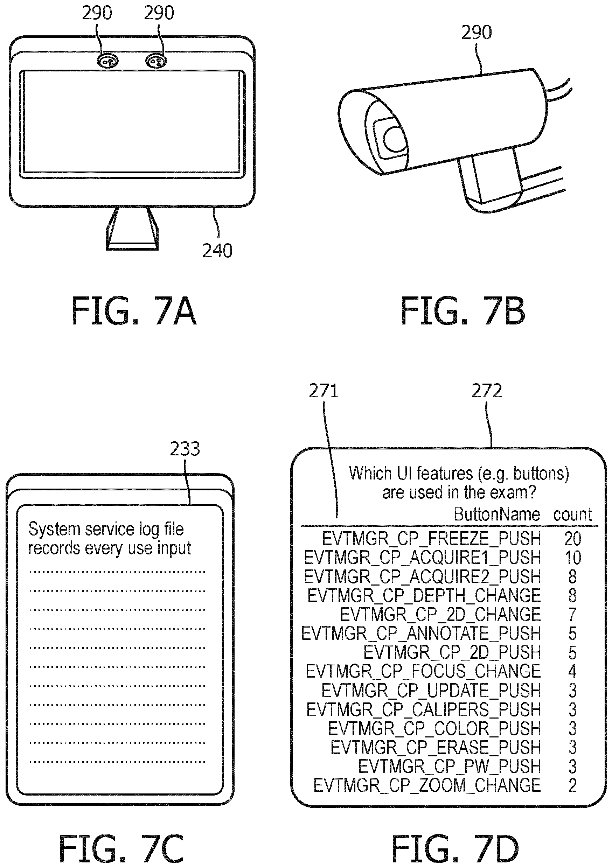

The disinfection classification module 260 also acquires ultrasound image data 232 from ultrasound images 510, 520, 530 during the exam (step 303). This ultrasound imaging data may include: user-selected system settings 513, 523,533, annotations 511, 521, 531 entered by a user during the exam, and organ locations 525 and interventional device locations 512, 532. System settings, such as probe and TSP data may be extracted from ultrasound images using natural language processing (step 307). The organs and interventional devices may be detected using information from the ultrasound exam order form 231 and deep learning methods, which are known in the art for organ and device detection. By detecting the organs and interventional devices in the ultrasound images, tracking data can be extracted from the ultrasound images (step 308) to inform disinfection classification based on contact determined by their relative positions. The annotations 511, 521, 531 are saved in the service log file 233 (shown in Fig. 7C), and can be extracted (step 306) and analyzed or processed using natural language to identify labels and body markers which may affect disinfection requirements during step 308. The annotations may also provide user observations that affect disinfection requirements, such as skin condition and leaks in a probe cover, for example.

The disinfection classification module 260 determines a recommended disinfection level using the extracted data 231, 232, 251 and deep learning (step 309). Deep learning trains an artificial intelligence algorithm of the disinfection classification module what data words or other data will affect the cleaning classification and how it will affect the cleaning classification. The disinfection classification module then provides post-exam decision data 261 as output to the disinfection guidance module 280. The post-exam decision data may comprise a Spaulding classification of the ultrasound exam: non-critical, semi-critical, critical and a required disinfection level: cleaning + LLD, cleaning + HLD, cleaning + sterilization.

Fig. 6 shows a process flow for the second building block of the invention - the contact tracking module 270. The contact tracking module 270 identifies surface areas of the ultrasound system that have been contacted by a user. This contact data is then used to provide guidance to the user for post-exam cleaning guidance.

The contact tracking module 270 acquires the system service log 233 (shown in Fig. 7C) from the imaging module 230 (step 601). Then, the contact tracking module 270 extracts contact data from the system service log 233 (step 603). Since each user's action is recorded in the system service log 233, the system service log provides a comprehensive record of contacts with soft keys and hard keys corresponding to the user's actions.

According to some embodiments, the contact tracking module 270 also acquires tracking video 291 from one or more cameras 290 (step 602). The cameras may be front facing cameras on the display 240 of the imaging system 200, as shown in Fig. 7A. The cameras may also be wall mounted cameras in the exam room as shown in Fig. 7B, or a combination of these cameras may be used. The contact tracking video 291 may be used to identify contacts with surfaces of ancillary equipment that does not have user interface sensing capability and would not show up in the system service log, such as ECG cables and possibly a scanner main monitor (display) (step 604). The contact tracking video can also help to identify which user has contacted a particular surface when more than one user is present during the exam. User contacts may be determined from the contact tracking video using image analysis techniques such as sectioning, or by tracking a user relative to known surface locations.

Next, the contact tracking module 270 generates an interface utilization report 272 from contact data 271 which are derived from the system service log 233 (step 605). The contact data may be augmented by the contact tracking video 291. The interface utilization report 272, shown in Fig. 7D provides a listing of surfaces (buttons) touched by a user during the exam.

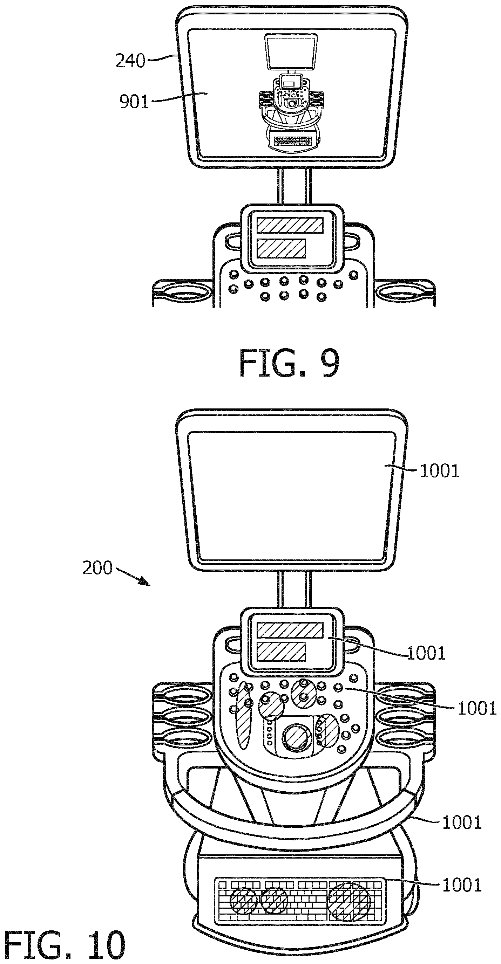

The contact tracking module 270 generates a heat map of user contacts (Step 606) highlighting surface areas that have been contacted, and therefore, need cleaning/disinfecting. The contact tracking module provides contact data 271 in the form of a heat map to the disinfection guidance module 280. The heat map may be an image of the ultrasound system presented on display 240 with contact areas 901 highlighted, as shown in Fig 9. Alternatively, the heat map may be provided by backlighting the actual surfaces 1001 that have been contacted, as shown in Fig. 10, provided that the ultrasound system has backlighting for user interface surfaces.

Fig. 8 shows a process flow for the third building block of the invention - a disinfection guidance module 280. The disinfection guidance module provides an alert and guidance to a user for post-exam disinfection and cleaning.

An ultrasound system user finishes an ultrasound exam (step 801). The exam may be finished by the user pressing an "end exam" button, for example. However, in different ultrasound systems, it is contemplated that other actions may be used to finish an ultrasound exam.

In response to the user finishing the exam, the disinfection guidance module 280 displays a disinfection alert 282 through an ultrasound system management module 201 to the display 240 (Step 802). The disinfection alert 285 (shown in Fig. 11) provides a recommended Spaulding disinfection level 1101, as determined by the disinfection classification module 260. In the illustrated example, the Spaulding disinfection level is "sterilization". The disinfection alert may be presented on a screen on display 240, and may be displayed instead of a new ultrasound exam order form, as currently is displayed upon finishing an exam on some ultrasound systems.

The disinfection alert 282 may include a "proceed to disinfection protocol" button 1102. In response to a user pushing the "proceed to disinfection protocol" button, the disinfection guidance module 280 provides visual guidance to the user for disinfection of the ultrasound system 200 (step 803). This guidance may include a heat map of surfaces that have been contacted during the exam and need to be cleaned and disinfected, as determined by the contact tracking module 270. Two alternative heat maps are shown in Figs. 9 and 10. The visual guidance may also include written directions and/or visual indications of cleaning and disinfecting products and equipment to be used based on the determined disinfection level, observations annotated during the exam, medical informatics, and the equipment (such as specific probe) used. The guidance may also include disinfection instructions and any other visual guidance that assists the user in performing proper cleaning and disinfection.

The disinfection alert 282 may further comprise a "done" button 1103 to be pushed by a user after completing the disinfection protocol to indicate that the disinfection protocol has been completed. While the "proceed to disinfection protocol" and "done" user interactions are described as buttons presented on the disinfection alert 285, they may also be presented in other formats within the scope of the invention, such as a pull down menu, for example.

According to one embodiment, the disinfection guidance module 280 freezes the ultrasound imaging system when the exam is finished (step 804), preventing the system from starting another exam until the disinfection protocol is completed.

The disinfection guidance module 280 may track an ultrasound transducer used during an exam by its serial number, as well as, tracking the time duration between pressing the "proceed to disinfection protocol" button and pressing the "done" button as a quality control measure to assure that the disinfection protocol has been performed. They may have an embedded Global Positioning Satellite (GPS) tracking sensor. If so, the position information (e.g. placed in a dedicated hydrogen peroxide mist station or liquid oak station for HLD) can also be integrated into the quality control process to verify compliance with the disinfection protocol.

If pre-exam information (e.g. patient medical record and ultrasound order form) is available on the ultrasound scanner connected to an ENR system, the invention can build an additional level of enforcement for pre-exam disinfection procedures. By analyzing the pre-exam clinical informatics, as well as the quality control level from the disinfection protocol of the previous exam, the system can alert the operator of the current exam to take necessary precautions to minimize cross-exam contamination, for example, covering the transducer with a protective sheath and using sterile gel for coupling.

The disinfection reminder and instructions can be archived in PDF, Word, or Excel format on the ultrasound system or on an institute file server to facilitate further data analysis and audit. Data filtering applied to searchable reports can create context-specific and actionable disinfection management reports based on the transducer and/or the operator.

The preceding description and accompanying drawings are intended to be illustrative and not limiting of the invention.
- 200: U/S imaging system
- 201: U/S system management module
- 210: processor
- 220: memory
- 230: U/S imaging module
- 231: U/S order form
- 232: U/S imaging data
- 233: System service log
- 240: display
- 250: medical record system
- 251: medical record
- 260: disinfection classification module
- 261: post-exam decision data
- 270: contact tracking module
- 271: contact data
- 272: interface utilization report
- 280: disinfection guidance module
- 282: disinfection alert
- 290: camera
- 300: method for determining an appropriate post-exam disinfection level
- 301: acquire medical record
- 302: acquire u/s exam order form
- 303: acquire U/S image data
- 304: extract medical data
- 305: extract procedure data
- 306: extract annotation data
- 307: extract probe and TSP data
- 308: extract tracking data
- 309: determine disinfection recommendations
- 401: U/S scanner
- 510: first U/S image
- 511: annotation
- 512: tracking data
- 513: user settings
- 520: Second U/S image
- 521: annotation
- 523: user settings
- 525: tracking data
- 530: third U/S image
- 531: annotation
- 532: tracking data
- 523: user settings
- 601: acquire system service log
- 602: acquire camera tracking video
- 603: extract contact data
- 604: track user contacts
- 605: generate user interface utilization report
- 606: generate heat map of user contacts
- 801: finish exam
- 802: display disinfection recommendation
- 803: provide visual guidance
- 804: freeze system
- 901: heat map image
- 1001: contact map
- 1101: disinfection alert window
- 1102: disinfection button
- 1103: done button

## Claims

1. A computer-implemented method (300) for identifying recommended post-exam disinfection and cleaning steps for an ultrasound system and providing an alert of said recommended disinfection and cleaning steps to a user of the ultrasound system, the method comprising the steps of:
obtaining clinical informatics, examination details, user selected ultrasound exam settings, and ultrasound images and annotations (301, 302, 303);
determining a recommended disinfection classification based on the clinical informatics, examination details, user selected ultrasound exam settings, and ultrasound images and annotations (308) and
providing an alert to a user to conduct a post exam disinfection procedure; the alert including the recommended disinfection classification (802).

2. The method of claim 1, wherein: the clinical informatics are obtained from an Electronic Health Record (EHR) system or an Electronic Medical Record (EMR) system, the examination details are obtained from an exam order form, and the user selected exam settings and ultrasound images and annotations are obtained from an ultrasound imaging module.

3. The method of claim 1, wherein the alert further includes a button for providing visual guidance for a disinfection protocol.

4. The method of claim 3, further comprising the steps of:
surveying a system service log file of the ultrasound system to determine user contact data for ultrasound system equipment surfaces; and
determining disinfection requirements for the ultrasound equipment system surfaces based on the user contact data;
wherein the visual guidance includes a heat map indicating user contacts with the ultrasound system equipment surfaces.

5. The method of claim 4, further comprising the step of analyzing video data from at least one camera to determine user contact data for ultrasound system equipment surfaces.

6. The method of claim 4, wherein the heat map is displayed as an image on a display of the ultrasound system.

7. The method of claim 4, wherein the heat map is displayed by backlighting contacted surfaces of the ultrasound system equipment.

8. The method of claim 1, wherein the alert includes a "proceed to disinfection" user interaction and a "done" user interaction, further comprising the step of provide disinfection protocol quality control by tracking an ultrasound transducer and a time duration between the timestamps for "proceed to disinfection protocol" and "done" user interactions.

9. An ultrasound system configured to identify recommended post-exam disinfection and cleaning steps for the ultrasound system and providing an alert of the recommended disinfection and cleaning steps to a user of the ultrasound system, the system comprising:
a processor (210)
a memory (220) operably connected to the processor; and
a medical record system (250) operably connected to the processor;
the memory having encoded thereon a program of instruction (201, 230, 260, 270, 280) executable by the processor to perform the steps of:
obtaining clinical informatics, examination details, user selected ultrasound exam settings, and ultrasound images and annotations (301, 302, 303);
determining a recommended disinfection classification based on the clinical informatics, examination details, user selected ultrasound exam settings, and ultrasound images and annotations (308) and
providing an alert to a user to conduct a post exam disinfection procedure; the alert including the recommended disinfection classification (802).

10. The ultrasound system of claim 9, wherein the alert further includes a button for providing visual guidance for a disinfection protocol.

11. The ultrasound system of claim 10, further comprising the steps of:
surveying a system service log file of the ultrasound system to determine user contact data for ultrasound system equipment surfaces; and
determining disinfection requirements for the ultrasound equipment system surfaces based on the user contact data;
wherein the visual guidance includes a heat map indicating user contacts with the ultrasound system equipment surfaces.

12. The ultrasound system of claim 11, wherein the heat map is displayed as an image on a display of the ultrasound system.

13. The ultrasound system of claim 11, wherein the heat map is displayed by backlighting contacted surfaces of the ultrasound system equipment.

14. A computer program product comprising a machine-readable storage media having encoded thereon program code for identifying recommended post-exam disinfection and cleaning steps for an ultrasound system and providing an alert of the recommended disinfection and cleaning steps to a user of the ultrasound system, comprising:
program code for obtaining clinical informatics, examination details, user selected ultrasound exam settings, and ultrasound images and annotations (301, 302, 303);
program code for determining a recommended disinfection classification based on the clinical informatics, examination details, user selected ultrasound exam settings, and ultrasound images and annotations (308) and
program code for providing an alert to a user to conduct a post exam disinfection procedure; the alert including the recommended disinfection classification (802).

15. The computer program product of claim 14, wherein the clinical informatics are obtained from an Electronic Health Record (EHR) system or an Electronic Medical Record (EMR) system, the examination details are obtained from an exam order form, and the user selected ultrasound exam settings and ultrasound images and annotations are obtained from an ultrasound imaging module.

## Patentansprüche

1. Computerimplementiertes Verfahren (300) zum Identifizieren empfohlener Desinfektions- und Reinigungsschritte nach einer Untersuchung für ein Ultraschallsystem und zum Bereitstellen einer Warnung über die empfohlenen Desinfektions- und Reinigungsschritte an einen Benutzer des Ultraschallsystems, wobei das Verfahren die folgenden Schritte umfasst:
Erhalten klinischer Informatik, Untersuchungsdetails, vom Benutzer ausgewählter Ultraschalluntersuchungseinstellungen sowie Ultraschallbilder und Anmerkungen (301, 302, 303);
Bestimmen einer empfohlenen Desinfektionsklassifizierung auf der Grundlage der klinischen Informatik, Untersuchungsdetails, vom Benutzer ausgewählten Ultraschalluntersuchungseinstellungen sowie Ultraschallbilder und Anmerkungen (308) und
Bereitstellen einer Warnung an einen Benutzer, eine Desinfektionsprozedur nach einer Untersuchung durchzuführen; wobei die Warnung die empfohlene Desinfektionsklassifizierung (802) beinhaltet.

2. Verfahren nach Anspruch 1, wobei die klinische Informatik aus einem elektronischen Gesundheitsdatensatzsystem (EHR) oder einem elektronischen Krankendatensatzsystem (EMR) erhalten werden, die Untersuchungsdetails aus einem Untersuchungsauftragsformular erhalten werden und die vom Benutzer ausgewählten Untersuchungseinstellungen und Ultraschallbilder und -nmerkungen aus einem Ultraschallbildgebungsmodul erhalten werden.

3. Verfahren nach Anspruch 1, wobei die Warnung weiter eine Schaltfläche zum Bereitstellen einer visuellen Anleitung für ein Desinfektionsprotokoll beinhaltet.

4. Verfahren nach Anspruch 3, das weiter die folgenden Schritte umfasst:
Durchsehen einer Systemdienstprotokolldatei des Ultraschallsystems, um Benutzerkontaktdaten für Geräteoberflächen des Ultraschallsystems zu bestimmen; und
Bestimmen des Desinfektionsbedarfs für die Geräteoberflächen des Ultraschallsystems auf Grundlage der Benutzerkontaktdaten;
wobei die visuelle Anleitung eine Wärmekarte beinhaltet, die Benutzerkontakte mit den Geräteoberflächen des Ultraschallsystems anzeigt.

5. Verfahren nach Anspruch 4, das weiter den Schritt des Analysierens von Videodaten von mindestens einer Kamera umfasst, um Benutzerkontaktdaten für Geräteoberflächen des Ultraschallsystems zu bestimmen.

6. Verfahren nach Anspruch 4, wobei die Wärmekarte als Bild auf einer Anzeige des Ultraschallsystems angezeigt wird.

7. Verfahren nach Anspruch 4, wobei die Wärmekarte durch Hintergrundbeleuchtung der Kontaktoberflächen des Ultraschallsystemgeräts angezeigt wird.

8. Verfahren nach Anspruch 1, wobei die Warnung eine Benutzerinteraktion "Mit der Desinfektion fortfahren" und eine Benutzerinteraktion "Erledigt" beinhaltet, wobei es weiter den Schritt umfasst, eine Qualitätskontrolle des Desinfektionsprotokolls durch Verfolgen eines Ultraschallwandlers und einer Zeitdauer zwischen den Zeitstempeln für die Benutzerinteraktionen "Mit dem Desinfektionsprotokoll fortfahren" und "Erledigt" bereitzustellen.

9. Ultraschallsystem, das dazu konfiguriert ist, empfohlene Desinfektions- und Reinigungsschritte nach einer Untersuchung für das Ultraschallsystem zu identifizieren und eine Warnung über die empfohlenen Desinfektions- und Reinigungsschritte an einen Benutzer des Ultraschallsystems bereitzustellen, wobei das System Folgendes umfasst:
einen Prozessor (210)
einen Speicher (220), der betriebsbereit mit dem Prozessor verbunden ist; und
ein medizinisches Aufzeichnungssystem (250), das betriebsbereit mit dem Prozessor verbunden ist;
wobei auf dem Speicher ein Befehlsprogramm (201, 230, 260, 270, 280) kodiert ist, das vom Prozessor ausführbar ist, um die folgenden Schritte durchzuführen:
Erhalten klinischer Informatik, Untersuchungsdetails, vom Benutzer ausgewählter Ultraschalluntersuchungseinstellungen sowie Ultraschallbilder und Anmerkungen (301, 302, 303);
Bestimmen einer empfohlenen Desinfektionsklassifizierung auf der Grundlage der klinischen Informatik, Untersuchungsdetails, vom Benutzer ausgewählten Ultraschalluntersuchungseinstellungen sowie Ultraschallbilder und Anmerkungen (308) und
Bereitstellen einer Warnung an einen Benutzer, eine Desinfektionsprozedur nach einer Untersuchung durchzuführen; wobei die Warnung die empfohlene Desinfektionsklassifizierung (802) beinhaltet.

10. Ultraschallsystem nach Anspruch 9, wobei die Warnung weiter eine Schaltfläche zum Bereitstellen einer visuellen Anleitung für ein Desinfektionsprotokoll beinhaltet.

11. Verfahren nach Anspruch 10, das weiter die folgenden Schritte umfasst:
Durchsehen einer Systemdienstprotokolldatei des Ultraschallsystems, um Benutzerkontaktdaten für Geräteoberflächen des Ultraschallsystems zu bestimmen; und
Bestimmen des Desinfektionsbedarfs für die Geräteoberflächen des Ultraschallsystems auf Grundlage der Benutzerkontaktdaten;
wobei die visuelle Anleitung eine Wärmekarte beinhaltet, die Benutzerkontakte mit den Geräteoberflächen des Ultraschallsystems anzeigt.

12. Ultraschallsystem nach Anspruch 11, wobei die Wärmekarte als Bild auf einer Anzeige des Ultraschallsystems angezeigt wird.

13. Ultraschallsystem nach Anspruch 11, wobei die Wärmekarte durch Hintergrundbeleuchtung der Kontaktoberflächen der Ultraschallsystemausrüstung angezeigt wird.

14. Computerprogrammprodukt, das ein maschinenlesbares Speichermedium umfasst, auf dem Programmcode zum Identifizieren empfohlener Desinfektions- und Reinigungsschritte nach einer Untersuchung für ein Ultraschallsystem codiert ist und zum Bereitstellen einer Warnung über die empfohlenen Desinfektions- und Reinigungsschritte an einen Benutzer des Ultraschallsystems, umfassend:
Programmcode zum Erhalten klinischer Informatik, Untersuchungsdetails, vom Benutzer ausgewählter Ultraschalluntersuchungseinstellungen sowie Ultraschallbildern und Anmerkungen (301, 302, 303);
Programmcode zum Bestimmen einer empfohlenen Desinfektionsklassifizierung auf der Grundlage der klinischen Informatik, Untersuchungsdetails, vom Benutzer ausgewählten Ultraschalluntersuchungseinstellungen sowie Ultraschallbilder und Anmerkungen (308) und
Programmcode zum Bereitstellen einer Warnung an einen Benutzer, nach einer Untersuchung eine Desinfektionsprozedur durchzuführen; wobei die Warnung die empfohlene Desinfektionsklassifizierung (802) beinhaltet.

15. Computerprogrammprodukt nach Anspruch 14, wobei die klinische Informatik aus einem elektronischen Gesundheitsdatensatzsystem (EHR) oder einem elektronischen Krankendatensatzsystem (EMR) erhalten werden, die Untersuchungsdetails aus einem Untersuchungsauftragsformular erhalten werden und die vom Benutzer ausgewählten Ultraschalluntersuchungseinstellungen und Ultraschallbilder und Anmerkungen aus einem Ultraschallbildgebungsmodul erhalten werden.

## Revendications

1. Procédé mis en oeuvre par ordinateur (300) pour l'identification d'étapes recommandées de désinfection et de nettoyage après examen pour un système d'échographie et de fourniture d'une alerte desdites étapes recommandées de désinfection et de nettoyage à un utilisateur du système d'échographie, le procédé comprenant les étapes de :
obtention d'informatique clinique, de détails d'examen, de paramètres d'examen échographique sélectionnés par l'utilisateur, et d'images échographiques et d'annotations (301, 302, 303) ;
détermination d'une classification de désinfection recommandée sur la base de l'informatique clinique, des détails d'examen, des paramètres d'examen échographique sélectionnés par l'utilisateur et des images échographiques et des annotations (308) ; et
fourniture d'une alerte à un utilisateur pour qu'il effectue une opération de désinfection après examen ; l'alerte incluant la classification de désinfection recommandée (802).

2. Procédé selon la revendication 1, dans lequel : l'informatique clinique est obtenue à partir d'un système de dossier de santé électronique (DSE) ou d'un système de dossier médical électronique (DME), les détails d'examen sont obtenus à partir d'un formulaire de demande d'examen, et les paramètres d'examen sélectionnés par l'utilisateur et les images échographiques et les annotations sont obtenus à partir d'un module d'imagerie échographique.

3. Procédé selon la revendication 1, dans lequel l'alerte inclut en outre un bouton pour fournir un guidage visuel pour un protocole de désinfection.

4. Procédé selon la revendication 3, comprenant en outre les étapes de :
étude d'un fichier journal d'entretien système du système d'échographie pour déterminer des données de contact avec l'utilisateur associées à des surfaces d'équipement de système d'échographie ; et
détermination d'exigences de désinfection pour les surfaces d'équipement de système d'échographie sur la base des données de contact avec l'utilisateur ;
dans lequel le guidage visuel inclut une carte thermique indiquant les contacts de l'utilisateur avec les surfaces d'équipement de système d'échographie.

5. Procédé selon la revendication 4, comprenant en outre l'étape d'analyse des données vidéo provenant d'au moins une caméra pour déterminer des données de contact avec l'utilisateur associées à des surfaces d'équipement de système d'échographie.

6. Procédé selon la revendication 4, dans lequel la carte thermique est affichée sous forme d'image sur un écran du système d'échographie.

7. Procédé selon la revendication 4, dans lequel la carte thermique est affichée par rétroéclairage des surfaces touchées de l'équipement de système d'échographie.

8. Procédé selon la revendication 1, dans lequel l'alerte inclut une interaction d'utilisateur « procéder à la désinfection » et une interaction d'utilisateur « terminé », comprenant en outre l'étape de fourniture d'un contrôle de qualité de protocole de désinfection en suivant un transducteur à ultrasons et une durée entre les horodatages associés aux interactions d'utilisateur « procéder au protocole de désinfection » et « terminé ».

9. Système d'échographie configuré pour identifier des étapes recommandées de désinfection et de nettoyage après examen pour le système d'échographie et fournissant une alerte des étapes recommandées de désinfection et de nettoyage à un utilisateur du système d'échographie, le système comprenant :
un processeur (210) ;
une mémoire (220) connectée de manière fonctionnelle au processeur ; et
un système de dossier médical (250) connecté de manière fonctionnelle au processeur ;
la mémoire présentant, codé sur celle-ci, un programme d'instructions (201, 230, 260, 270, 280) exécutable par le processeur pour exécuter les étapes de :
obtention d'informatique clinique, de détails d'examen, de paramètres d'examen échographique sélectionnés par l'utilisateur, et d'images échographiques et d'annotations (301, 302, 303) ;
détermination d'une classification de désinfection recommandée sur la base de l'informatique clinique, des détails d'examen, des paramètres d'examen échographique sélectionnés par l'utilisateur et des images échographiques et des annotations (308) ; et
fourniture d'une alerte à un utilisateur pour qu'il effectue une opération de désinfection après examen ; l'alerte incluant la classification de désinfection recommandée (802).

10. Système d'échographie selon la revendication 9, dans lequel l'alerte inclut en outre un bouton pour fournir un guidage visuel pour un protocole de désinfection.

11. Procédé selon la revendication 10, comprenant en outre les étapes de :
étude d'un fichier journal d'entretien système du système d'échographie pour déterminer des données de contact avec l'utilisateur associées à des surfaces d'équipement de système d'échographie ; et
détermination d'exigences de désinfection pour les surfaces d'équipement de système d'échographie sur la base des données de contact avec l'utilisateur ;
dans lequel le guidage visuel inclut une carte thermique indiquant les contacts de l'utilisateur avec les surfaces d'équipement de système d'échographie.

12. Système d'échographie selon la revendication 11, dans lequel la carte thermique est affichée sous forme d'image sur un écran du système d'échographie.

13. Système d'échographie selon la revendication 11, dans lequel la carte thermique est affichée par rétroéclairage des surfaces touchées de l'équipement de système d'échographie.

14. Produit de programme informatique comprenant un support de stockage lisible par machine présentant codé sur celui-ci un code de programme pour identifier des étapes recommandées de désinfection et de nettoyage après examen pour un système d'échographie et fournir une alerte des étapes recommandées de désinfection et de nettoyage à un utilisateur du système d'échographie, comprenant :
un code de programme pour obtenir une informatique clinique, des détails d'examen, des paramètres d'examen échographique sélectionnés par l'utilisateur, et des images échographiques et des annotations (301, 302, 303) ;
un code de programme pour déterminer une classification de désinfection recommandée sur la base de l'informatique clinique, des détails de l'examen, des paramètres d'examen échographique sélectionnés par l'utilisateur et des images échographiques et des annotations (308) ; et
un code de programme pour fournir une alerte à un utilisateur pour qu'il effectue une opération de désinfection après examen ; l'alerte incluant la classification de désinfection recommandée (802).

15. Produit de programme informatique selon la revendication 14, dans lequel l'informatique clinique est obtenue à partir d'un système de dossier de santé électronique (DSE) ou d'un système de dossier médical électronique (DME), les détails d'examen sont obtenus à partir d'un formulaire de demande d'examen, et les paramètres d'examen échographique sélectionnés par l'utilisateur, les images échographiques et les annotations sont obtenus à partir d'un module d'imagerie échographique.
